# EUROPEAN PATENT APPLICATION

(11) **EP 3 696 819 A1**
(43) Date of publication of application: **19.08.2020**
(21) Application number: 19156936.7
(22) Date of filing: 13.02.2019
(51) Int. Cl.: G16H 30/00, G16H 40/60, G16H 50/00

(54) **CALCULATION OF DIAGNOSTIC SIMILARITY BETWEEN DIFFERENT MR PROTOCOLS**

(71) Applicant: Siemens Healthcare GmbH, 91052 Erlangen (DE)
(72) Inventor: Blum, Christian, 91077 Neunkirchen am Brand (DE); Blum, Thomas, 91077 Neunkirchen am Brand (DE)

(57) **Abstract**

The present invention relates to the calculation of a similarity between two or more MR protocols (P) to be used on a MR scanner (MR) by means of a classifier (100). The classifier (100) has been trained to provide a classification result (R) in form of classifying a received protocol (P) with respect to its relevance for a given medical question (Q).

## Description

The present invention refers to the domain of medical imaging and in particular to an automatic, machine-implemented approach to classify protocols for an MRI apparatus with respect to their medical meaning.

In a typical clinical setting, usually several medical imaging apparatuses, like e.g. MR scanners, are used and operated in parallel. A MR scanner is operated with a set of different protocols for different medical questions.

Magnetic resonance imaging (MRI) combines high magnetic fields, magnetic field gradients, and radio frequency excitation pulses to generate and spatially encode magnetic resonances in a human patient or other imaging subject. The magnetic resonances are processed by a Fourier transform or other reconstruction process to decode the spatial encoding and produce a reconstructed image of the subject. Depending on the diagnostic question, different operating protocols are to be applied.

Further, MR apparatuses may vary in their constructional features so that they are adapted to examine different body parts or different tissue or organs. The protocol may be represented by a set of so called protocol parameters. For understanding the relevance of specific protocol parameters (e.g. TE, TR, slice thickness / distance / orientation, flip angle) for a certain medical question (e.g. head cancer, lung cancer, heart disease, etc.) a detailed level of understanding of both medical background knowledge and technological and constructional knowledge of the apparatus is necessary. During the course of medical development and research, oftentimes new protocol versions or new protocols are to be rolled out on a set of apparatuses. Thus, an 'existing' set of protocols needs to be exchanged by an updated version thereof. This, however, makes it necessary to evaluate the equivalence or medical similarity between the updated version and the existing (old) one. This task is error prone, because it is important that no information is lost and that no functional protocol is deleted or overwritten by a similar but not diagnostically identical protocol.

The task of distributing protocols onto a family of scanners is a difficult task which oftentimes makes it necessary that a medical assistant evaluates similarity of two or more protocols manually.

In state of the art it is known to provide protocol trees. Thus, a particular patient protocol may be selected from a "User Protocol Tree" that is typically stored in and accessed through the MR scanner. Generally, the MR scanner is operated by extracting (or defining from appropriate data storage) MR protocol parameters for the patient's MR imaging examination. Further, the protocol parameters may be updated for e.g. the patient's age or to get a certain image representation and quality resulting in changes being made to the MR image pulse sequence parameters that affect image contrast. For example, the patient's age may be used to select the appropriate expected MR relaxation times of the patient tissue under examination. The optimum repetition times (TR), inversion times (TI), and/or echo times (TE) for T1 weighted, T2 weighted, or proton density images are then calculated by use of the well known Bloch equations. An optimized MR protocol parameter set is derived from the calculations and used in the MR protocol where contrast between two tissues (for example, gray matter and white matter in a neuroimaging application) may be maximized.

This process is time consuming and error prone as huge sets of protocol parameters have to be evaluated and a high degree of expert knowledge is necessary.

It is therefore an object of the present invention to improve security and quality of protocol evaluation. In particular, distributing MR protocols onto a set of MR scanners should be automated and accelerated. Further, an automatic comparison of a first protocol with a second protocol with respect to medical similarity on a diagnostic content basis and not on a parameter level should be provided. Finally, it is an object of the present invention to provide a method and an apparatus which avoid the necessity of employing a manual comparison of protocol parameters.

The object, mentioned above, is achieved by a method, a classifier, an MR apparatus and a computer program according to the appended independent claims. Advantageous aspects, features and embodiments are described in the dependent claims and in the following description together with further advantages.

According to a first aspect the present invention relates to a computer-implemented method for classifying one or more operating protocol(s), e.g. MR protocols, to be used on an imaging apparatus (e.g. MR apparatus), wherein the method comprises:
- Providing a memory with a computer-based classifier (which may comprise a supervised or unsupervised computer-implemented learning module) which has been trained to provide a classification result for an (new, updated version or unannotated) operating protocol with respect to its diagnostic relevance for a medical question; the classification result may comprise to provide protocol clusters for its diagnostic relevance for a set of different medical questions;
- Receiving an (unannotated, unknown/new) operating protocol to be classified with respect to its diagnostic relevance for a given medical question;
- Accessing the classifier in the memory for using or applying the classifier for the received operating protocol as input for providing the classification result for the received operating protocol. The classification result may be binary (part of the classification cluster or not) or may comprise a multi-element result, e.g. an output vector. The learning module may comprise an artificial neural network (ANN) or a support vector machine (SVM).

The advantage of the proposed approach is to reduce the number of protocols, stored on an MR scanner. Further, the distribution of new updates and protocol versions may be improved and simplified. No evaluation of protocols on a parameter level is necessary. Protocols may be grouped and clustered automatically according their diagnostic relevance for a certain given medical question (head cancer, abdominal cancer, heart disease etc.). Further, the technical administration of protocols (e.g. electronic access) becomes significantly faster and easier as less protocols are to be stored and thus be compared to new updates. In case of protocol similarity, e.g. an existing or old protocol may be overwritten by an update version, which has been evaluated to be similar without loss of information. Storing the old version is no longer necessary.

According to a preferred embodiment, the method may be used for comparing a first operating protocol with a set of second operating protocols (wherein the set of second protocols may only consist of one single protocol) with respect to its diagnostic relevance for a given medical question in order to assess a protocol similarity on a content basis and not on a protocol parameter basis. This has the advantage, that similarity may be assessed on a much more transparent level and by taking into account the medical use case and the diagnostic question.

In another preferred embodiment of the present invention, the classifier is a computer-implemented or machine learning module and comprises a trained artificial neural network (in short ANN) or a support vector machine (in short: SVM). By means of the machine learning module it is possible to automatically classify new protocols or new or updated or otherwise amended protocol versions into clusters, which are categorized according to their medical content or medical meaning. As a consequence, the operation of the MR device may be made significantly safer and more secure than the traditional approach, because the appropriate protocols may be evaluated on a medical content level. This also means, that it is safer (smaller error rate) for a physician or medical personal to evaluate the protocols from a medical, clinical or diagnostic point of view. It gets thus easy to get knowledge about which protocol is suitable and appropriate for which medical question (e.g. "Is there an image-based evidence for lung cancer?" or "Is there an image-based evidence for a heart disease?" etc.) without going into details of different protocol parameters and their interdependencies.

In another preferred embodiment of the invention, the classifier has been trained by a supervised learning method with annotated operating protocols, wherein the annotations indicate the diagnostic relevance of the respective protocol for a given medical question. Thus, it is necessary to provide correct input/output pairs, wherein the input generally relates to the protocol and the output refers to the medical question or its relevance for the medical question. The network is trained by calculating the error between a target result (output cluster in form of the medical question) and the actual ANN output. A correct neural network answer will be learned with these training data in order to be able to classify new (un-annotated) protocols with respect to their medical appliance correctly. This feature is based on the fact that a huge amount of annotated training data exists already and is accessible via an interface, e.g. a DICOM interface.

In another alternative embodiment, the classifier may be trained by an unsupervised learning method. This has the advantage that it is not necessary to provide annotated protocol data. A data driven or manual evaluation partitions the set of protocols in appropriate clusters according to their medical question, which may be answered with the protocol.

In yet another embodiment, it is possible to use a reinforcement learning (learning from failure). It differs from supervised learning in that correct input/output pairs need not be presented. The classification results of the ANN are evaluated and the reinforcing results (rewards and/or penalties) are fed back to the ANN. This improves quality of the classification result.

In a preferred example embodiment, the proposed solution relates to MR technology. Thus, the operating protocol may be an MR protocol and the imaging apparatus may be an MR apparatus. However, alternatively, the principles of the present inventions, may also be applied to other imaging technologies, like CT, molecular imaging, x-ray or others.

In a preferred embodiment, the provided classification result may be forwarded to a second classifier, which is configured to classify the provided classification result into a second classification result, which has a higher level of detail compared to the classification result. It is also possible to provide a sequence of classifiers providing a sequence of higher detail level. Thus, the additional classifiers may be connected or wired in series to provide more detailed classification levels. The level of detail may for example relate to: "detection of pathological tissue in the breast, classification of liver / brain lesions, detection of prostate cancer - etc.".

According to another preferred embodiment, the provided classification result is forwarded to a validation unit, which comprises a bidirectional (e.g. graphical) user interface for providing the classification result and for receiving a validation signal. The validation signal may be a user interaction, like switching or clicking a button or a control. The validation signal may be provided to the classifier. This has the advantage that the significance of the classification result may be improved, because an expert may control and monitor the classification result manually, which may lead to another self-learning and adaption of the classifier, which in the end improves quality of the classification task.

According to another preferred embodiment, the method is used in a protocol update procedure. By using the classifier, it is possible that only selection of updated protocols is stored on the imaging apparatus in addition to existing protocols, wherein the selection is automatically calculated by having the same or a similar classification result as the existing protocols with respect to its diagnostic relevance. The technical advantage of this aspect is that the number of protocols may be reduced, which makes errors due to problematic protocol constellations and determinations less likely. Thus, errors may be reduced, the stack of stored protocols may be controlled and administered in an improved manner and the process of selecting a specific protocol on a scanner by the medical staff (selected from the set of stored protocols) may be simplified. Less storage capacity is necessary.

When applying the method in a protocol update procedure, an unintended and erroneous protocol deletion (of an existing protocol by overriding) may be excluded so that the update procedure will be more secure and safe. In particular, it may be configured that only those protocols from the group of existing protocols are subject to an overriding procedure (and thus subject to deletion) which are evaluated as having the same or a similar classification result as the update protocol with respect to its diagnostic relevance. This has the technical effect that a protocol may only be overwritten by a similar protocol, so that it may be assured that no information is lost. Thus, only a selection of updated protocols - namely similar protocols - are stored on the imaging apparatus.

According to another preferred embodiment, the method is used in a protocol distribution procedure for distributing a given operating protocol on other or different imaging apparatuses. By means of a distribution module which is configured to execute a distribution algorithm, a given operating protocol - which is subject to distribution - is only implemented and/or stored on selected imaging apparatuses. The selection of imaging apparatuses is calculated by the distribution algorithm, which accesses the classifier for finding those apparatuses on which identical or similar protocols are stored. Thus, it may be assured that the given protocol is only installed on those devices, which are operated with protocols which have the same of a similar classification result as the given imaging protocol. This reduces errors and makes the protocol control and technical administration (the protocols are processed automatically by algorithms so that they may be subject to manual evaluation) easier.

Up to now, the invention has been described with respect to the method. Features, advantages or alternative embodiments herein can be assigned to the other claimed objects (e.g. the classifier or the imaging device as instances of an apparatus claim, the computer program or a computer program product) and vice versa. In other words, claims for the apparatuses can be improved with features described or claimed in the context of the methods. In this case, the functional features of the method are embodied by structural units of the computer system or apparatus and vice versa, respectively. This is due to the dual character of software, which may be embodied as a hardware unit (an integrated circuit unit like an ASIC or FPGA etc.) or as a software unit. For a person skilled in the art it is clear that the functional features may be embodied in respective hardware modules; for example, the process of protocol distribution may be embodied in a distribution unit and the process of protocol update and storage may be embodied in an update unit and in a memory.

In another aspect the invention refers to a classifier for classifying an operating protocol to be used on an imaging apparatus, wherein the classifier has been trained to provide a classification result for an operating protocol (usually being new and unannotated) with respect to its diagnostic relevance for a given medical question, comprising:
- An input interface for receiving the operating protocol;
- An output interface for providing the classification result. The classification result is automatically calculated without the need for manual user interaction and on the basis of and with respect to a given medical question. The calculations for the classification of the classifier are thus based on the medical or diagnostic content, relevance and/or meaning. The calculations are not based on protocol parameters. This has the advantage that only medical knowledge and no or less physical knowledge about the imaging devices is necessary.

In a preferred embodiment, the classifier may be configured with a protocol interface to a protocol memory, in which a set of existing protocols is stored (protocol stack), which may be used and applied for the imaging procedure, if the imaging device is operated with the selected protocol from the stack of protocols.

In a preferred embodiment, the classifier may be configured with an interface for detecting the medical question (as given medical question) of which the classification task should be based. The interface may be integrated into the input interface.

In another aspect the invention refers to an imaging apparatus with a classifier, as described above.

In another aspect the invention refers to computer program which may be embodied on a computer readable medium or which may be provided for download from a server instance (e.g. cloud based) for classifying an operating protocol to be used on an imaging apparatus, comprising program instructions, which when executed on a computer, cause the computer to perform a method as mentioned above, In particular, the computer program may comprise:
- logic for the classifier;
- access to an input interface for receiving an operating protocol to be classified; and
- access to an output interface for providing the classification result;
- optionally an additional interface for receiving a given medical question, so that the received operating protocol is classified with respect to its diagnostic relevance for the given medical question in order to provide the classification result.

In another aspect, the invention relates to a computer program product comprising a computer program, the computer program being loadable into a memory unit of a computing unit, including program code sections to make the computing unit execute the method for classification as described above, when the computer program is executed in said computing unit.

In another aspect the invention relates to a computer-readable medium, on which program code sections of a computer program are stored or saved, said program code sections being loadable into and/or executable in a computing unit to make the computing unit execute the method for classification as described above, when the program code sections are executed in the computing unit.

The realization of the invention by a computer program product and/or a computer-readable medium has the advantage that already existing devices like computing units for an e.g. imaging apparatus (CT, MRT,...), which may act as clients can be easily adopted by software updates in order to work as proposed by the invention.

In the following a definition of terms used within this application is given.

The term "classifying" relates to the classification task, performed by the classifier which is exclusively executed automatically by means of a computer and an algorithm executed thereon. In particular, protocols for operating an imaging device, like MR protocols are classified with respect to their diagnostic or medical meaning and in particular to a medical question to be answered by an image, obtained by the imaging device, being operated with the protocol.

The classification task being executed by the classifier access a classification module, which may be a software module. The classifier may be implemented in various ways.

In a first embodiment, the classifier may be an artificial neural network (ANN), which is trained with a machine-learning technique. The training data may be annotated data, i.e. protocols which are annotated with respect to the respective medical question they could answer. The architecture of the ANN may be a network with different hidden layers (to provide a fine-grained classification result). Different learning methods may be used, including supervised and unsupervised learning. Generally, the algorithms involved with an ANN are able to capture complex, nonlinear, relationships in high dimensional feature spaces. However, ANNs are based on the Empirical Risk Minimization (ERM) principle. Therefore, they are prone to false optimizations due to local minima in the optimization function and are susceptible to training problems such as "overfitting." This makes ANN-training a complex procedure.

In recent years, Support Vector Machines (SVMs) have been found to be remarkably effective in many real-world applications. Unlike ANNs, SVMs follow the Structural Risk Minimization (SRM) principle, which aims at minimizing an upper bound of the generalization error. As a result, an SVM tends to perform well when applied to data outside the training set. SVMs also have many desirable properties such as flexibility in choice of kernel function and implicit mapping into high dimensional feature spaces. But what makes SVMs most attractive is that they avoid several major problems associated with ANNs. For example, SVMs control overfitting by restricting the capacity of the classifier. They also depend on the solution of a convex Quadratic Programming (QP) problem which has no local extrema. The unique optimal solution can therefore be efficiently obtained.

Thus, in a second embodiment, the classifier may be implemented as support vector machine (SVM). SVM method originally used in statistical learning theory, has now been developed and applied to various classification and regression problems. SVM may well have to solve the problem of sparse training data set, and the use of high-dimensional feature space to solve the non-linear classification. Another benefit is that the objective function SVM is the convex hull, the obtained local optimal SVM usually global optimal solution.

The result of the classification mentioned above is the classification result, which may be binary (yes, no), which represents the protocol being or not being a representative of a protocol cluster. The classification result may also be more complex and comprise different clusters according to different medical and/or diagnostic questions, which may be evaluated with the protocol. For example, the classification result may be a set of clusters like "head - T1", "head - T2", "head - general", "heart - general" etc.

The term "medical question" is a dataset which represents medical or diagnostic content, e.g. a medical question can be "is there a tumor in a certain brain structure?" Examples for medical questions are: unclear headache, abdomen pain, whole body trauma, breast cancer screening etc.

The operating protocol is a set of instructions and settings (e.g. with a set of parameters) for operating an imaging device, like an MR apparatus, CT apparatus a positron emission tomography (PET) device etc. and thus may be an MR protocol, a CT or a PET protocol etc. Generally, in positron emission tomography, a positron-emitting radiopharmaceutical is administered to a human patient or other imaging subject. Each positron annihilation produces two oppositely directed gamma rays having energies of about 511 keV. The two gamma rays are detected by radiation detectors surrounding the imaging subject, defining a line of response (LOR). A multitude of such positron annihilation events define projection-like LOR data that can be reconstructed e.g. by filtered backprojection, iterative reconstruction, or another reconstruction technique to produce a reconstructed image.

The training data may be annotated data which comprise an input output pair of a protocol and a target classification result.

The computer for executing the classification task, mentioned above, may be a personal computer or a workstation in a computer network and may include a processing unit, a system memory, and a system bus that couples various system components including the system memory to the processing unit. The system bus may be any of several types of bus structures including a memory bus or memory controller, a peripheral bus, and a local bus using any of a variety of bus architectures. The system memory may include read only memory (ROM) and/or random access memory (RAM). A basic input/output system (BIOS), containing basic routines that help to transfer information between elements within the personal computer, such as during start-up, may be stored in ROM. The computer may also include a hard disk drive for reading from and writing to a hard disk, a magnetic disk drive for reading from or writing to a (e.g., removable) magnetic disk, and an optical disk drive for reading from or writing to a removable (magneto) optical disk such as a compact disk or other (magneto) optical media. The hard disk drive, magnetic disk drive, and (magneto) optical disk drive may be coupled with the system bus by a hard disk drive interface, a magnetic disk drive interface, and a (magneto) optical drive interface, respectively. The drives and their associated storage media provide nonvolatile storage of machine readable instructions, data structures, program modules and other data for the computer. Although the exemplary environment described herein employs a hard disk, a removable magnetic disk and a removable (magneto) optical disk, those skilled in the art will appreciate that other types of storage media, such as magnetic cassettes, flash memory cards, digital video disks, Bernoulli cartridges, random access memories (RAMs), read only memories (ROM), and the like, may be used instead of, or in addition to, the storage devices introduced above. A number of program modules may be stored on the hard disk, magnetic disk, (magneto) optical disk, ROM or RAM, such as an operating system, one or more application programs, like the method for classifying and/or other program modules, and/or program data for example. A user may enter commands and information into the computer through input devices, such as a keyboard and pointing device, for example. Other input devices such as a microphone, joystick, game pad, satellite dish, scanner, or the like may also be included. These and other input devices are often connected to the processing unit through a serial port interface coupled to the system bus. However, input devices may be connected by other interfaces, such as a parallel port, a game port or a universal serial bus (USB). A monitor (e.g. a GUI) or other type of display device may also be connected to the system bus via an interface, such as a video adapter for example. In addition to the monitor, the computer may include other peripheral output devices, such as speakers and printers for example.

The computer may operate in a networked environment which defines logical connections to one or more remote computers. The remote computer may be another personal computer, a server, a router, a network PC, a peer device or other common network node, and may include many or all of the elements described above relative to the personal computer. The logical connections include a local area network (LAN) and a wide area network (WAN), an intranet and the Internet.

The properties, features and advantages of this invention described above, as well as the manner they are achieved, become clearer and more understandable in the light of the following description and embodiments, which will be described in more detail in the context of the drawings. This following description does not limit the invention on the contained embodiments. Same components or parts can be labeled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

Fig. 1 is a schematic overview representing general aspects and a structural overview and architecture of the classifier according to a preferred embodiment.
Fig. 2 is an embodiment of the classifier and
Fig. 3 shows another embodiment of the classifier and
Fig. 4 also shows other possible embodiments of the classifier.
Fig. 5 is a schematic overview figure of an MR apparatus with a classifier and
Fig. 6 shows an embodiment of the classifier being applied in a protocol update process with a validation unit.
Fig. 7 is a flow chart of a classification method according to a preferred embodiment of the present invention.

### Detailed description of the embodiments

The present invention relates to machine-based evaluation and classification of operating protocols (in the following also shortly named as 'protocol') for an imaging device, in particular an MR scanner which is referenced with numeral MR in the figures. The classification aims at comparing MR protocols on a content basis and not on the basis of MR protocol parameters, which necessitates sound knowledge about the physical capabilities of the MR scanner physics.

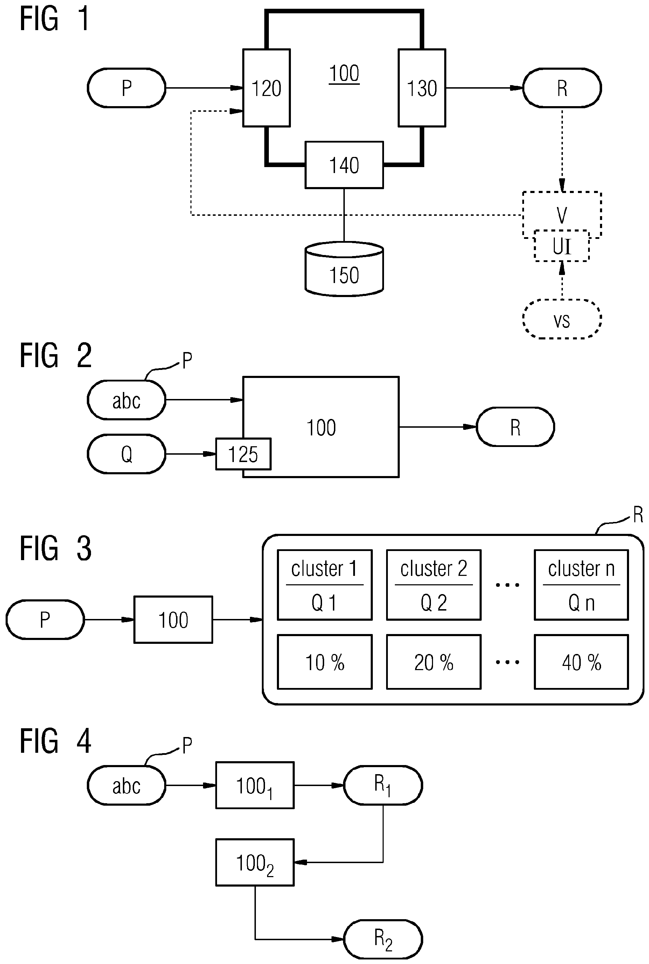

The classification task is performed by a classifier 100, which - as can be seen in Fig. 1 - is configured with an input interface 120 for receiving an operating protocol P (in the following also named: MR protocol) and with an output interface 130 for providing the classification result R. Optionally, the classifier may also comprise a protocol interface 140 for accessing a protocol memory which is adapted to store a set of MR protocols which may be executed on the MR scanner. Alternatively, the protocol memory 150 may be part of a computer which is provided for operating and controlling the MR scanner MR.

It is optionally possible to provide a validation unit 200, which is equipped with a user interface UI for receiving a validation result vs. The validation result vs may be fed back to the classifier 100 for improving classification quality and for applying a self-learning procedure on the classifier 100. In Fig. 1, the validation unit V is depicted in dotted lines, because it is optional and the classifier 100 can also be operated without the validation unit V.

Fig. 2 shows a first embodiment of the classifier 100, where the classification result R is provided in a binary structure, comprising two result cases or clusters YES and NO. This embodiment is especially beneficial when the classification is to be performed for a particular given medical question Q. The medical question Q may for example be "Is there an evidence for a cardiac valvular insufficiency?" The classifier 100 may comprise a question interface 125 for receiving the given medical question. Generally, the input interface 120 and the question interface 125 may be integrated in one common (e.g. user) interface. In this embodiment, the classifier 100 is configured to evaluate whether or not the given protocol P (in Fig. 2, named abc) may be appropriate for examining the patient with respect to the given medical question, received via interface 125 ("valvular insufficiency?"). The classification result R may be YES or NO.

Fig. 3 shows another option for calculating or providing the classification result R. In this embodiment, the result R is more complex and comprises a set of classification clusters or groups. The classification clusters represent a probability that the given protocol P is suitable and may be used for answering a respective medical question to a certain degree. In the example, shown in Fig. 3, the medical question Q1 in cluster 1 may be used for diagnosing the medical question 1 to 10%, the medical question Q2 in cluster 2 may be used for diagnosing the medical question 2 to 20%, the medical question n in cluster n may be used for diagnosing the medical question n to 40%. In this example case, n=3 and only three cluster classes are used. The amount of clusters is depending on the complexity of the classifier 100 and the diversity of medical questions Q.

In a preferred embodiment, shown in Fig. 4, two classifiers 100₁, 100₂ are wired in sequence. The first classifier 100₁ may e.g. be trained for classifying a given input protocol P into clusters of body regions, e.g. head, spine, abdomen, etc. The second classifier 100₂ may e.g. be trained for classifying the very given input protocol (which has been subject to be provided to the first classifier 100₁) only within the first result R₁ - provided by the first classifier 100₁ - and thus within the appropriate cluster or classification class, which has been calculated by the first classifier 100₁. The second classifier 100₂ may then provide a more detailed answer for which kind of medical questions in the result cluster R₁ the given protocol P is appropriate, e.g. for which kind of head diseases the protocol P may be applied and executed correctly. In this embodiment, the first classifier 100₁ is trained to cluster a given protocol P into coarse body regions or organs, whereas the second classifier 100₂ is trained to cluster the given protocol P within such a body region or first result cluster, provided by the first classifier 100₁. In this case the classification task of the second classifier is to cluster protocols into a set of clusters, wherein the number of the set of (output) clusters corresponds to the number of results, provided by the first classifier. For example, if the first classifier provides a classification into only three body regions head, spine, abdomen, then the second classifier needs to execute three different classification tasks, namely a mapping to the first, second or third result cluster of the first classifier. In an alternative embodiment, it is also possible to provide different classifiers for the different classification tasks on the second level and thus for all of the first result cluster classes.

Generally and in other embodiments, more than two classifiers 100 may be connected or wired in series to provide a sequence of classifiers, such as the first result R1 of the first classifier 100₁ is fed into a second classifier 100₂ for providing a second result R₂, which again may be subject to be provided as input to a third classifier 100₃ for providing a third result R₃ and so on and so forth. The sequence of classifiers 100 may for example provide more detailed results for more detailed medical questions.

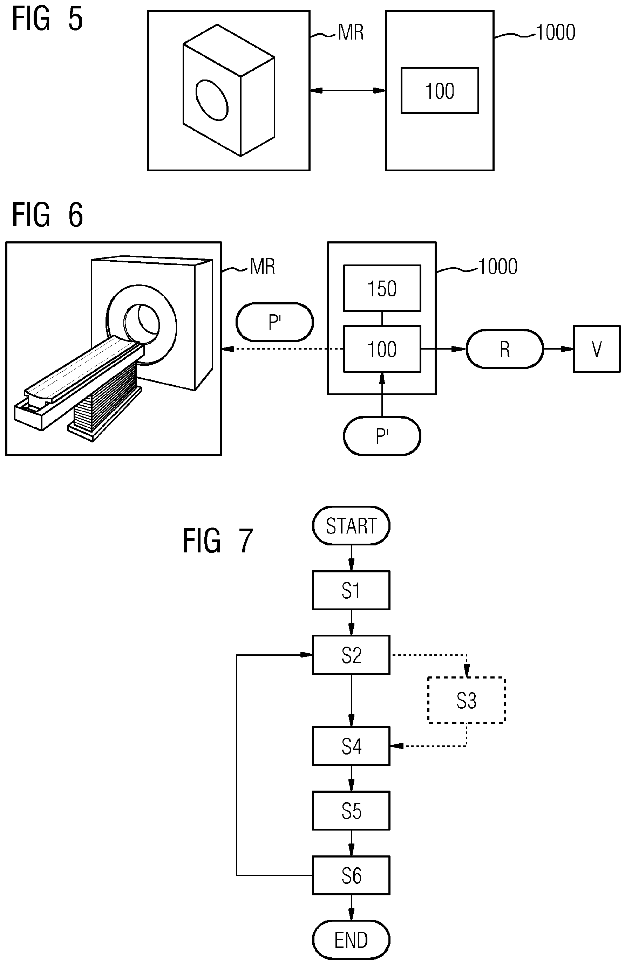

Fig. 5 shows an overview of an example architecture for a classifier 100 within a medical domain. The MR apparatus MR is controlled and operated by means of control software, being deployed on a computer 1000. The computer 1000 is e.g. responsible for controlling and/or parameterizing the connected MR scanner hardware, controlling the examination work-flow, providing the user interface information and/or reconstructing the image data and/or can consist of one or several dependent or independent computers.

On the computer 1000, the classifier 100 is installed. The computer 1000 may also comprise the protocol memory 150, in which a set of working protocols are stored. The computer 1000 may in addition be equipped with an input interface. The input interface 120 and/or the output interface 130 of the classifier 100 may be provided on the computer 1000. The validation unit V may also be provided on the computer 1000 or can be deployed on any computer system of a cloud installation. In an alternative embodiment, the computer 1000 is not provided as a single separate unit (as shown in Fig. 5) but is deployed on a computing unit of the MR apparatus MR itself. The classifier 100 implemented on the computer 1000 may then be directly and locally accessed on the MR apparatus MR. The computer 1000 is provided with a CPU to execute a software routine for the classification method as described above. In particular, the computer program or software routine is configured to comprise:
- the logic for the classifier 100;
- an input interface 120 for receiving an operating protocol P to be classified with respect to its diagnostic relevance for a given medical question Q; the medical question Q may also be received via an interface or determined by other means;
- an output interface 130 for providing the classification result R for the received operating protocol P.

Fig. 6 shows an example embodiment of the classifier 100 within an update procedure. A given updated protocol P' should be evaluated on an MR apparatus MR only if the classifier 100 provides a classification result R which indicates that the update protocol P' is classified in the same or similar classification cluster as the existing protocols which are stored in the memory 150 to be accessed for being used for the imaging procedure on the scanner MR. Optionally, a validation signal vs may be evaluated for control of the update procedure. As shown in Fig. 6 with the dashed line, the update protocol P' is only provided to the MR scanner MR if the classifier 100 verifies the update protocol P' as being similar or identical with respect to the medical questions Q to be answered.

Fig. 7 is a flow chart of the classification method according to the invention. After start of the method, in step S1 the trained classifier 100 is provided. It may be trained in a preceding training phase in order to be able to classify any given (new, unannotated, unknown) protocol P with respect to its diagnostic relevance for a medical question Q. In step. S2 a protocol P is received via an input interface 120, which needs to be classified. In an embodiment, an optional step S3 may be executed (shown in Fig. 7 with dotted lines) in which a specific medical question Q is received. In another embodiment, the medical question which should be subject to the classification task is determined by other means. In a first embodiment, the medical question Q is determined by reading a dataset out from a memory, e.g. from the MR scanner MR. In a second embodiment, the medical question Q may be determined in a preparation phase. It may be fixed or static. In a third embodiment, the medical question Q may be a dataset with a set of questions. It may be determined in the execution phase, e.g. by user input. Both, the medical question Q as well as the protocol P to be classified are forwarded to the classifier 100 for the classification task. In step S4 the classifier 100 is accessed for be applied or used in step S5 for the received protocol P to be classified for its relevance to a given medical question Q. In step S6 the classification result R is calculated by the classifier 100 and may be provided on the output interface 130. Further measures may be applied as mentioned before, like the validation by the validation unit V and/or another classification task for the result R or a feedback loop of the provided result R to the classifier 100 for being improved. After this, the method may end or may be re-iterated.

The different embodiments are example embodiments. Generally, a single unit, module or device may fulfil the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantageous which are described with respect to a particular embodiment of present invention or with respect to a particular figure are, wherever applicable, also advantages of other embodiments of the present invention.

## Claims

1. Computer-implemented method for classifying an operating protocol (P) to be used on an imaging apparatus (MR), wherein the method comprises:
- Providing (S1) a memory (150) with a computer-based classifier (100) which has been trained to provide a classification result (R) for a given operating protocol (P) with respect to its diagnostic relevance for a medical question (Q) ;
- Receiving (S2) an operating protocol (P) to be classified with respect to its diagnostic relevance for the given medical question (Q);
- Accessing (S4) the classifier (100) for using (S5) the classifier (100) for the received operating protocol (P) for providing (S6) the classification result (R).

2. Method according to claim 1, wherein the method is used for comparing a first operating protocol (P1) with a set of second operating protocols (P2) with respect to its diagnostic relevance for the given medical question (Q) in order to assess a protocol similarity on a content basis and not on a protocol parameter basis.

3. Method according to any of the preceding claims, wherein the classifier (100) is a machine learning module and comprises a trained artificial neural network (ANN) or a support vector machine (SVM).

4. Method according to any of the preceding claims, wherein the classifier (100) has been trained by a supervised learning method with annotated operating protocols, wherein the annotations indicate the diagnostic relevance for a medical question.

5. Method according to any of the preceding claims 1 to 3, wherein the classifier (100) has been trained by an unsupervised learning method.

6. Method according to any of the preceding claims, wherein the operating protocol (P) is an MR protocol and the imaging apparatus is an MR apparatus (MR).

7. Method according to any of the preceding claims, wherein the provided classification result (R) is forwarded to at least one second classifier (100₂), which is configured to classify the provided classification result (R) into at least one second classification result (R₂), which has a higher level of detail compared to the classification result (R).

8. Method according to any of the preceding claims, wherein the provided classification result (R) is forwarded to a validation unit (V), which has a user interface (UI) for displaying the classification result (R) and for receiving a validation signal (vs) and wherein the validation signal (vs) is provided to the classifier (100) for adaption.

9. Method according to any of the preceding claims, wherein the method is used in a protocol update procedure, such as only selection of updated protocols (P') is stored on the imaging apparatus (MR) in addition to existing protocols (P), wherein the selection is automatically calculated by determining those update protocols (P') having the same or a similar classification result (R) as the existing protocols (P') with respect to its diagnostic relevance.

10. Method according to any of the preceding claims, wherein the method is used in a protocol update procedure, such as only those protocols from the group of existing protocols (P) are subject to an overriding within the update procedure which have been evaluated as having the same or a similar classification result (R) as the update protocol (P') with respect to its diagnostic relevance.

11. Method according to any of the preceding claims, wherein the method is used in a protocol distribution procedure for distributing a given operating protocol (P') on different imaging apparatuses (MR₁, MR₂, ...MRₙ), so that the given operating protocol (P') is only stored on those selected imaging apparatuses, which are operated with existing protocols (P) which have the same of a similar classification result (Q) as the given imaging protocol (P').

12. Classifier (100) for classifying an operating protocol (P) to be used on an imaging apparatus (MR), wherein the classifier (100) has been trained to provide a classification result (R) for an operating protocol with respect to its diagnostic relevance for a given medical question (Q), comprising:
- An input interface (120) for receiving the operating protocol (P);
- An output interface (130) for providing the classification result (R) for the received operating protocol (P) with respect to its diagnostic relevance for the given_medical question (Q).

13. Classifier (100) according to the directly preceding claim, wherein the classifier (100) is configured with a protocol interface (140) to a protocol memory (150), in which a set of existing protocols (P) is stored.

14. Imaging apparatus (MR) with a classifier (100) according to any of the preceding claims, directed to the classifier.

15. Computer program embodied on a computer readable medium for classifying an operating protocol (P) to be used on an imaging apparatus (MR), comprising program instructions, which when executed on a computer (1000), cause the computer (1000) to perform a method according to any of the preceding method claims.
